# EUROPÄISCHE PATENTSCHRIFT

(11) **EP 1 050 064 B1**
(45) Veröffentlichungstag und Bekanntmachung des Hinweises auf die Patenterteilung: **26.03.2003**
(21) Anmeldenummer: 98966654.0
(22) Anmeldetag: 21.12.1998
(51) Int. Cl.: H01J 37/32

(54) **IMPLANTATION VON RADIOAKTIVEN ?32 P-ATOMEN**
IMPLANTATION OF RADIOACTIVE ?32 P ATOMS
IMPLANTATION D'ATOMES DE P?32 RADIOACTIFS

(30) Priorität: 24.12.1997 DE 19757852
(43) Veröffentlichungstag der Anmeldung: 08.11.2000
(73) Patentinhaber: Forschungszentrum Karlsruhe GmbH, 76133 Karlsruhe (DE)
(72) Erfinder: FRIEDRICH, Ingeborg, D-76137 Karlsruhe (DE); HUTTEL, Erhard, D-76344 Eggenstein-Leopoldshafen (DE); KALTENBAEK, Johann, D-76297 Stutensee (DE); SCHLÖSSER, Klaus, D-76344 Eggenstein-Leopoldshafen (DE)
(74) Vertreter: Rückert, Friedrich, Dr.
(86) Internationale Anmeldenummer: EP9808379
(87) Internationale Veröffentlichungsnummer: WO99034396

(56) Entgegenhaltungen:
- US-A- 3 778 656
- US-A- 4 778 561
- US-A- 4 780 642
- US-A- 4 992 665
- US-A- 5 607 442

## Beschreibung

Die Erfindung betrifft eine Elektron-Zyklotron-Resonanz-Ionen-Quelle, (Electron-Cyclotron-Resonance-Ion-Source) ECRIS, und ein Verfahren zum Betreiben dieser ECRIS. Die ECRIS findet z.B. in einer Anlage zur Herstellung von Gefäßstützen (Stents), die in einer solchen Anlage mit radioaktiven und nichtradioaktiven Atomen und Molekülen dotiert werden, Anwendung.

Solche Gefäßstützen, in der Fachsprache Stents genannt, werden in der Medizin zur Therapie von Gefäßstenosen verwandt. Hierzu werden sie mit einem radioaktiven Element dotiert, das sich in seiner Halbwertszeit an der Therapiezeit orientiert, d. h. im wesentlichen die Heilungszeit einer operativen Verletzung im Körper ins Kalkül nimmt. Es wird damit Restenosen vorgebeugt.

Da die Halbwertszeit ein verhältnismäßig kurzer Zeitraum ist - 14 Tage ist als solcher anzusetzen - ist eine Lagerhaltung dieser Stents nicht möglich. Vielmehr müssen diese Stents quantifizierbar - und dies reproduzierbar - mit dem geeigneten radioaktiven Element dotiert werden, wobei in die Überlegung der Restradioaktivität beim Einsetzen in den menschlichen Körper der Transportweg und die Transportzeit von der Dotierung bis zur Implantation mit in die Vorgabewerte einzubeziehen sind.

Damit stellt sich die Frage des optimalen Bestrahlungsverfahrens und die Frage nach der Anlage, mit der eine solche Bestrahlung zuverlässig reproduzierbar durchgeführt werden kann.

Eine solche Anlage besteht aus drei zentralen Komponenten, nämlich der Ionenquelle mit Extraktionssystem, der Massenseparationseinrichtung in Form eines Dipolmagneten und der Bestrahlungskammer. Von Elektron-Zyklotron-Resonanz-Ionenquellen ist bekannt, daß sie bei großer Ausbeute Ionenstrahlen mit hoher Qualität liefern. Daher ist eine solche Ionenquelle Bestandteil einer solchen Anlage. Der Massenseparator ist ein Dipolmagnet, der den extrahierten Ionenstrahl beim Durchlaufen massenspezifisch auffächert. In der Bestrahlungskammer schließlich wird der exponierte Gegenstand dem ausgewählten Teilstrahl zur Dotierung ausgesetzt. Die Beschickung und Entnahme des Gegenstands oder der Gegenstände aus der Bestrahlungskammer geschieht, indem die Bestückungskammer mit Argon geflutet und dann geöffnet wird.

ECR-Ionenquellen werden u. a. in dem Handbuch "Ion Sources", herausgegeben von Bernhard Wolf, 1995, CRC Press Boca Raton, New York, London, Tokyo, auf den Seiten 488-516 überblickartig mit weiterführenden Hinweisen erläutert. Die prinzipielle Arbeitsweise der ECRIS wird in diesem Buch u. a. in dem Abschnitt 9.2 "Working Principle of the Ion Source and Description of the Discharge" auf den Seiten 122 bis 127 erläutert. Ausführlicher, ins Detail gehend, ist das Buch "Electron Cyclotron Resonance Ion Sources and ECR Plasmas" von R. Geller, Institut of Physics Publishing, Bristol and Philadelphia, 1996. In letzterem sind neben dem Kapitel 2. 4, Antennen und Koppelstrukturen, die Kapitel 5, " Simple Mirror ... ", und 6, Min-B ECRIS for ... ", sehr ausführlich insbesondere auch hinsichtlich des Quellenaufbaus.

ECR-Quellen wurden zuerst hauptsächlich zur Erzeugung hochgeladener Ionen verwendet, wozu man Mikrowellen höherer Frequenzen braucht. Bei Anwendung niedriger Frequenzen bleibt der Vorteil der hohen Ionenausbeute immer noch erhalten, auf die es hier hauptsächlich ankommt. Ökonomisch bietet die Anwendung niedriger Mikrowellenfrequenzen den Vorteil, daß es bei 2,45 GHz preiswerte Mikrowellengeneratoren gibt, und außerdem vergleichsweise niedrige Magnetfelder erforderlich sind. Schwierig wird jedoch bei diesen niedrigen Frequenzen die Ankopplung der Mikrowellen an das Plasma, wenn die Wellenlänge größer als die Dimensionen der Plasmakammer wird.

Der Erfindung liegt die Aufgabe zugrunde, eine zuverlässige ECR-Ionenquelle zu konstruieren, die ein ausgesprochen gutes Langzeitverhalten zeigt, so daß in der Bestrahlungskammer stets ein genutzte Teilstrahl mit konstanter Qualität aus einem massenspezifisch gut aufgefächerter Ionenstrahl reproduzierbar für die Dotierung auf einen in der Kammer exponierten Stent oder auf mehrere, gleichzeitig exponierte Stents auftrifft.

Die Aufgabe wird mit einer ECRIS gemäß den Merkmalen des Anspruchs 1 und dem daran betriebenen Verfahren gemäß den Merkmalen des Anspruchs 7 gelöst.

Die Mikrowellenleistung wird über ein Koaxialkabel, das im Bereich der ECRIS parallel zu deren Achse zwischen zwei beliebigen, benachbarten Polen (Anspruch 2) verläuft, zugeführt.

Der die Plasmabrennkammer umschließende Sechspolmagnet- für den radialen Einschluß befindet sich in einem dielektrischen Rohr und isoliert damit das jeweils in den beiden Endbereichen der Plasmabrennkammer koaxial zur Achse umliegende Solenoid (Anspruch 3) Das verbessert die Hochspannungssicherheit wesentlich. Die Folie im Rohr hat den Vorteil, daß auf der Innenfläche des dielektrischen Rohres keine elektrostatischen Feldspitzen auftreten können, wie sie ohne sie durch die scharfkantige Struktur des Sechspols entstehen würden. Da die Folie keine leitende Verbindung zu dem Sechspol hat, wird sie durch Sprühentladung an den Kanten so aufgeladen, daß die Feldstärken, die zu dieser Entladung führen, teilkompensiert werden und dadurch die Sprühentladung wieder mitreduziert (Anspruch 4).

Plexiglas (Anspruch 5) ist.durchsichtig und läßt daher sehr gut Veränderungen oder Schäden erkennen, wie sie sich durch elektrischen Stress oder Wärmestress ausbilden.

Die Plasmakammer ist ein (doppelwandiges) Pyrexrohr. Auf diese Weise kann die Mikrowellenleistung von allen Seiten radial einkoppeln. Ein kleiner Rekombinationskoeffizient ermöglicht einen Neutralgasanteil mit hohem Wasserstoffatomanteil. Nur im Atomzustand ("naszierender Wasserstoff") ist Wasserstoff chemisch aggressiv und reagiert mit dem an der Wand kondensierten Phosphor zu gasförmigem Phosphorwasserstoff (Anspruch 6).

Sinnvoll ist der Dauerstrichbetrieb der Quelle. Unvorteilhaft wäre der getaktete Betrieb - der grundsätzlich möglich wäre - da während der Zeit, wenn die Ionenquelle abgeschaltet ist, immer noch Verluste an radioaktivem Phosphorwasserstoff entstünden.

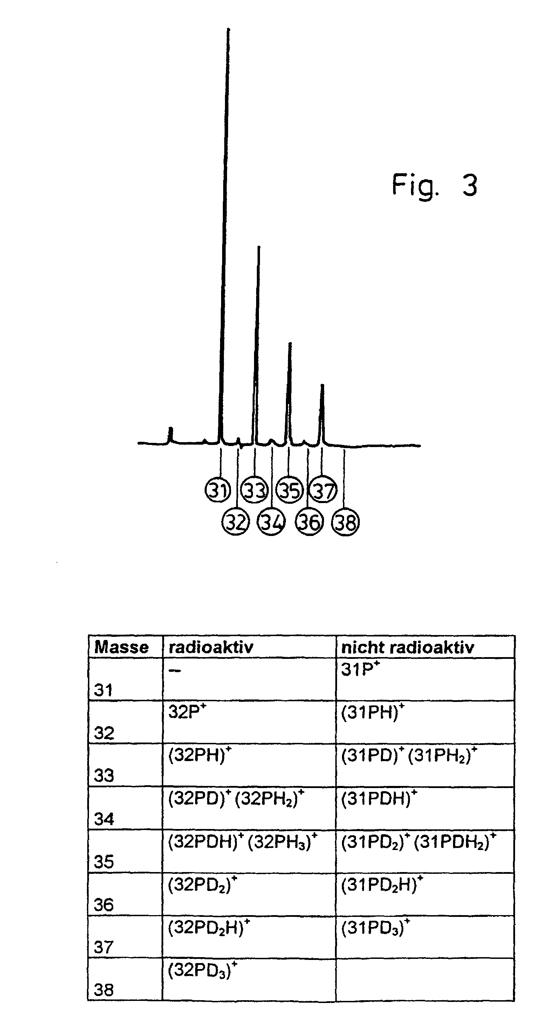

In Anspruch 7 ist das grundsätzliche Verfahren zum Betreiben der ECRIS beschrieben. Grundsätzlich muß es gehalten sein, da, wie später noch anhand der Figur 3 erläutert wird, ein beliebiges Ausblenden eines Teilstrahls aus einem Gesamtstrahl je nach Gasund Zusatzgasbefüllung sowie dem Einstellen der elektrischen Betriebsparameter an der ECRIS und dem Separationsmagneten und der Expositionsposition der Stents möglich sein soll und ist.

Ein hervorzuhebender elektrischer Parameter ist die Einstellbarkeit der Hochspannung an der ECRIS für die Extraktion

Der radioaktive Stent ist die mit einen aus der mit dem Verfahren betriebenen ECRIS ausgewählten Teilstrahl beaufschlagte Gefäßstütze. Die zusätzliche Implantation von nicht radioaktiven Atomen verändert die Stents auf günstige Weise indem vorzeitiges Auswaschen während der Positionierung und in der Endposition vermieden wird.

Die ECR-Ionenquelle liefert einen genügend energiereichen Ionenstrahl, dessen Nutzanteil tief genug in die Stents eindringt. So beträgt die Energie z. B. 60 keV. Die Mikrowellenführung beansprucht keinen Raum, der nicht schon vom Sechspol beansprucht wird. Ein weiterer Vorteil der Anlage besteht darin, daß dem Strahl, der mit dem Separationsmagneten (Dipol) präpariert wird, definiert zusätzlich Ionen, beispielweise geeignete Molekülionen, beigemischt werden, die zusammen mit den radioaktiven und nichtradioaktiven Atomen in die Gegenstände implantiert werden, wodurch die Qualität derselben bezüglich Korrosion und Auswaschung der Radioakivität verbessert wird.

Die Erfindung wird anhand der Zeichnung im folgenden näher erläutert. Die Zeichnung besteht aus den Figuren 1 bis 3 und einer Tabelle. Es zeigt:
Figur 1 den schematische Aufbau der Anlage,
Figur 2 den schematischen Aufbau der ECR-Ionenquelle,
Figur 3 das Massenspektrum der ECR-Ionenquelle für radioaktiven Phosphor und die Tabelle die möglichen Massenanteile.

Die Ionenquelle arbeitet mit geringen Substanzmengen von radioaktivem Phosphor. Etwa 5 mg natürlicher Phosphor (³¹P) ist mit 10⁻¹ radioaktivem Phosphor (³²P) angereichert. Ein möglichst hoher Anteil der radioaktiven Phosphoratome, die in die Ionenquelle gebracht werden, soll in den durch den Separationsmagneten 2 abgetrennten hochenergetischen Strahl aus Atom- und Molekülionen übergehen. Die Ionenquelle 1 liefert einen Ionenstrahl, von dem durch den Separationsmagneten 2 ein Nutzanteil zugänglich gemacht wird, der einen genügend großen Anteil an radioaktiven Phosphoratomen oder Phosphormolekülen enthält, damit die für die Anwendung notwendige Dosis in dem Stent oder den Stents implantiert werden kann.

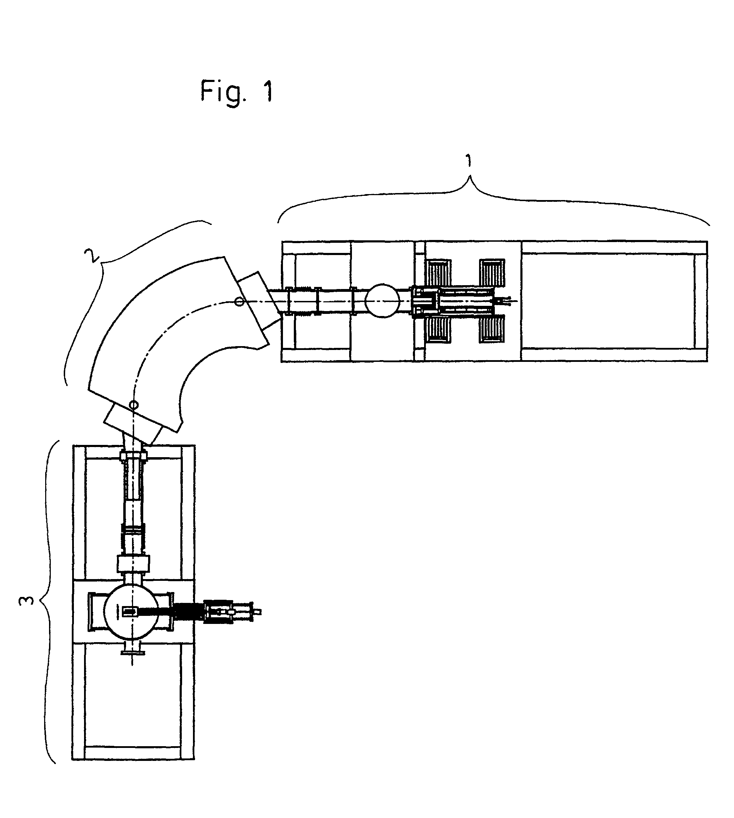

Die Anlage besteht aus den drei Komponenten: Elektron-Zyklotron-Resonanz-Ionen-Quelle 1, Separationsmagnet 2 und Bestrahlungskammer (3 Figur 1). Der Ionenstrahl wird mit der ECR-Ionenquelle 1 erzeugt und in Richtung des Separationsmagneten 2 daraus extrahiert, indem er beim Durchlauf massenspezifisch aufgefächert und präpariert wird. Die Exponate in der Bestrahlungskammer 3, die Gefäßstützen oder Stents, sind so aufgestellt, daß der Nutzanteil des Ionenstrahls, der aus hochenergetischen radioaktiven Phosphorionen oder nicht radioaktiven Phosphormolekülionen besteht, auf dieselben trifft und als radioaktive oder nichtradioaktive Phosphoratome in das Strukturmaterial implantiert werden.

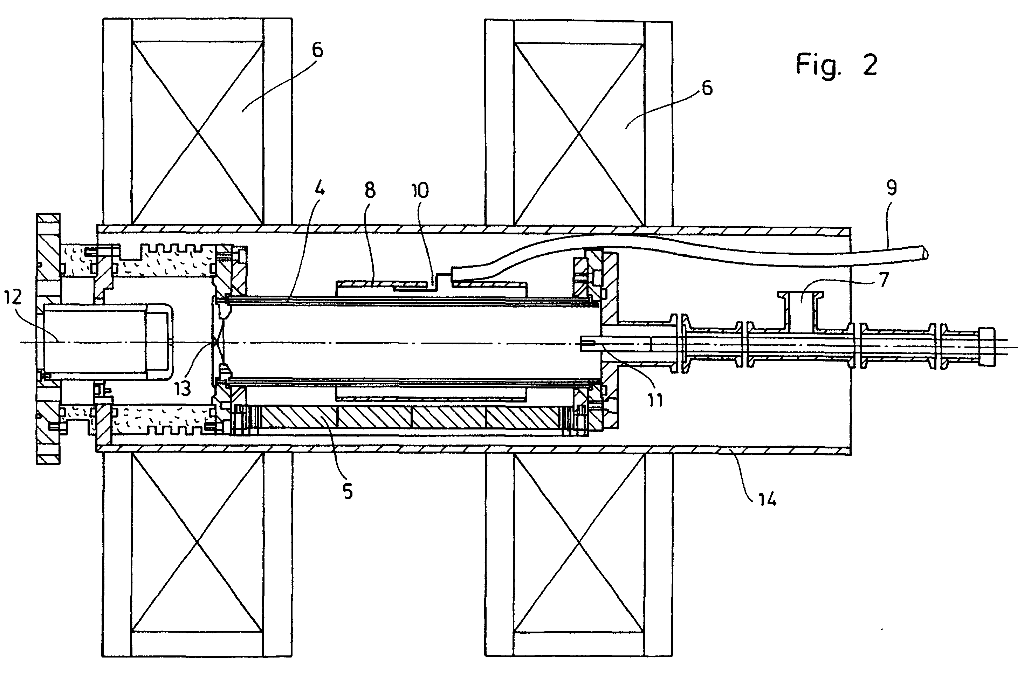

Figur 2 zeigt die ECR-Ionenquelle im axialen Schnitt. Die zylindrische Plasmabrennkammer 4, deren Wände für Mikrowellen transparent sind und deren Wandmaterial einen möglichst kleinen Rekombinationskoeffizienten hinsichtlich der Molekülbildung aus Atomen des Zusatzgases hat, befindet sich konzentrisch in dem Sechspol-Permanentmagneten 5. Über dessen beiden und der Plasmabrennkammer beiden Enden sind die magnetischen Spiegelspulen 6 angebracht. Über einen Gaseinlaß 7 wird das geeignete Gas, beispielsweise Wasserstoff oder Deuterium in die Plasmabrennkammer eingelassen. Dieses Gas wird durch die in die Kammer 4 eingekoppelte Mikrowelle ionisiert. Das magnetische Sechspolfeld und das axiale magnetische Spiegelfeld bewirken, daß die bei der Ionisation gebildeten Elektronen nur schwer die Wand der Plasmabrennkammer 4 erreichen. Dadurch entsteht eine große Dichte an Elektronen, die, von den Mikrowellen vermöge der Elektron-Zyklotron Resonanz angetrieben, eine Pendelbewegung in der Plasmabrennkammer 4 ausführen. Dadurch wird ein sehr hoher Ionisationsgrad des in der Plasmabrennkammer 4 befindlichen Gases erreicht.

Zur Einkopplung der Mikrowelle dient ein dünnwandiges Rohrstück 8, das konzentrisch zwischen der Plasmabrennkammer 4 und dem Sechspol-Permanentmagneten 5 untergebracht wird und axial zur Optimierung der Mikrowellenanpassung hin und her verschoben werden kann. Die Mikrowelle wird über ein dünnes Koaxialkabel 9 dem Rohrstück 8 zugeführt. Hierzu kontaktiert der Außenmantel des Koaxialkabels 9 das Rohrstück 8 an der Einkoppelöffnung 10 und endet dort. Der Innenleiter wird isoliert durch diese Öffnung 10 nach innen geführt. Der isolierte Innenleiter ist zu einer parallel zur Mittelachse 12 gerichteten Schleife geformt und kontaktiert an der Innenwand des Rohrstücks 8. Über diese Schleife wird die Mikrowelle in das Rohrstück 8 eingekoppelt. Hierdurch ergeben sich zwei Vorteile:
i. Durch die axiale Verschiebemöglichkeit des Rohrstücks 8 wird die Schwierigkeit bei der Ankopplung der Mikrowellen an das Plasma umgangen, die dadurch entsteht, daß die Wellenlänge größer als die Gefäßdimensionen ist, und sich die Mikrowellenleistung nicht genügend in der Plasmabrennkammer ausbreitet. Man kann nämlich das Rohrstück 8 so verschieben, daß die maximale Feldstärke der Mikrowellen im Bereich der magnetischen Resonanzfeldstärke liegt.
ii.Man kommt mit so wenig Mikrowellenleistung aus, daß man sie mit einem dünnen Koaxialkabel parallel zur Achse der Ionenquelle 1 zwischen den Sechspolen 5 zuführen kann. Durch diese in keiner Weise den Ionenquellenaufbau störende Mikrowellenzuführung kann die gesamte ECR-Ionenquelle mit Ausnahme der Spiegelspulen 6 durch ein dielektrisches Rohr 14, hier ein Plexiglasrohr, von den Spiegelspulen 6 elektrisch isoliert gehalten und damit problemlos mit 60 kV oder darüber betrieben werden. Damit ist man in der Energieeinprägung des Ionenstrahls in weiten Grenzen frei.

Der radioaktive Phosphor wird in den Halter 11 eingepaßt und in der Ionenquelle plaziert. Durch die Wechselwirkung mit dem Plasma, das mit Wasserstoff- oder Deuteriumgas betrieben wird, geht der Phosphor in dieses über und wird fast unmittelbar auf der Wand der Plasmabrennkammer 4 abgeschieden.

Einen Vorteil bietet die Verwendung von H₂ oder D₂ zur Erzeugung des Plasmas, wenn die Innenwand der Plasmabrennkammer 4 aus Pyrex ist, das einen niedrigen Rekombinationskoeffizienten hinsichtlich der Molekülbildung aus Wasserstoff- oder Deuteriumatomen hat. Dann nämlich erhält man eine hohe Dichte von Wasserstoff- bzw. Deuteriumatomen und Ionen in der Plasmabrennkammer, die chemisch mit dem auf der Innenwand niedergeschlagenen Phosphor reagieren. Dabei entstehen gasförmige Phosphorwasserstoff- verbindungen, die in den Reaktionsraum zurückfallen und Phosphor wieder in das Plasma zurückführen.

Die ECR-Ionenquelle wird bei sehr niedrigem Neutralgasdruck und hohem Ionenanteil betrieben. Zur Ionenextraktion ist auf der Achse 12 ein nur kleines Loch 13 in der Plasmabrennkammer 4 angebracht. Daher verliert die ECR-Ionenquelle nur wenig neutrale Atome oder Moleküle. Der Phosphor verläßt hauptsächlich entweder als Atomion oder als Molekülion der verschiedenen Phosphorwasserstoffmoleküle im extrahierten, hochenergetischen Ionenstrahl, hier z. B. 60 keV, die Ionenquelle. Die Anwendung des chemischen Verfahrens ist vorteilhaft für die Erfüllung der Forderungen, mit geringen radioaktiven Substanzmengen auszukommen und als hinreichend mit radioaktiven Ionen angereicherter und energiereicher Ionenstrahl den Separationsmagneten zu verlassen.

Figur 3 zeigt den Ausschnitt aus dem Massenspektrum, wenn die ECR-Ionenquelle mit radioaktivem Phosphor betrieben wird. Die Betriebsprameter sind dabei folgende: Die Ionenquelle wird mit D₂ betrieben. Es werden 5 mg ³¹P zugesetzt, der mit einem Anteil von etwa 10⁻⁵ mit radioaktivem ³²P angereichert ist. Bekannt ist, daß Phosphor bis zu drei H- bzw. D-Atome binden kann. Grundsätzlich können in dem Plasma auch Phosphormolekülionen gebildet werden, die nur ein oder zwei Wasserstoff- bzw. Deuteriumatome enthalten. Die Massen entsprechen neben den Ionen der reinen Atome ³¹P und ³²P einer vielfältigen Kombination von Phosphorwasserstoff- bzw. Phosphordeuteriumionen. Die vollständige Tabelle zeigt alle in Frage kommenden Kombinationen, getrennt nach radioaktiven und nicht radioaktiven Phospor- und Phosphorwasserstoffionen. Es treten die Massenzahlen von 31, normaler Phosphor, bis 38, vollständig deuteriertes, radioaktives Phosphin, auf. Die Prototypquelle liefert regelmäßig 30 µA nichtradioaktive ³¹P⁺-Ionen.

Aus der Tabelle ergeben sich folgende Konsequenzen:
- Wird der Anteil normalen Wasserstoffs im Plasma ausreichend niedrig gehalten und wird der Separationsmagnet auf die Masse 32 eingestellt, kann immer ein Strahl erzeugt werden, der genügend Anteile an radioaktivem Phosphor enthält, um die für die Medizin benötigten Stents ausreichend mit radioaktivem Phospor, ³²P, zu dotieren.
- Wird die Quelle mit reinem Deuterium (D₂) betrieben und wird gezielt Wasserstoff (H₂) beigemengt, wird mit ein und demselben Strahl radioaktiver Phosphor und nichtradioaktiver Phosphor implantiert. Dadurch wird die Qulität des bestrahlten Gegenstands, des Stents, bezüglich Korrosion und Auswaschung der Radioaktivität verbessert.

### Bezugszeichenliste:

- 1: Elektron-Zyklotron-Resonanz-Ionenquelle, ECR-Ionenquelle
- 2: Separationsmagnet, Dipolmagnet
- 3: Bestrahlungskammer
- 4: Plasmabrennkammer
- 5: Sechspol-Permanentmagnet
- 6: Spiegelspule
- 7: Gaseinlaß
- 8: Rohrstück
- 9: Koaxialkabel
- 10: Einkoppelöffnung
- 11: Halter, Phosphorhalter
- 12: Achse, Mittelachse
- 13: Extraktionsöffnung
- 14: Rohr

## Patentansprüche

1. Elektron-Zyklotron-Resonanz-Ionenquelle (Electron-Cyclotron-Resonance-Ion-Source = ECRIS) (I) mit Ionenextraktionseinrichtung, für eine Anlage zur Dotierung von Gefäßstützen mit radioaktiven und nichtradioaktiven Atomen, mit einer Plasmabrennkammer (4), einem konzentrisch um die Plasmabrennkammer angeordnete magnetischen Sechspal (5) und Mittels zum Einkoppeln einer Mikrowellenleistung in die Plasma brennkammer (4),
**dadurch gekennzeichnet, daß**
sich zum Einkoppeln der Mikrowellenleistung ein dünnwandiges und elektrisch leitendes Rohrstück (8) koaxial zwischen der zylindrischen Plasmabrennkammer (4) und dem magnetischen Sechspol (5) befindet und auf der Plasmabrennkammer (4) zur Einstellung der optimalen Mikrowelleneinkopplung axial verschiebbar ist,
ein Koaxialkabel (9), mit dem die Mikrowellenleistung zu einer Einkoppelöffnung (10) in der Wand des Rohrstücks (8) heran geführt wird, dort mit seinem Außenmantel elektrisch leitend kontaktiert, der Innenleiter isoliert durch die Einkoppelöffnung (10) ragt und an der Rohrinnenwand eine flache Schleife bildet, deren Ende wiederum auf der Rohrinnenwand kontaktiert,
die Plasmabrennkammer (4) mikrowellendurchlässig ist.

2. ECRIS nach Anspruch 1,
**dadurch gekennzeichnet, daß**
das Koaxialkabel (9) für die Mikrowellenzuführung parallel zur Achse (12) der Plasmabrennkammer (4) zwischen zwei Polen des Sechspols (5) verlegt ist.

3. ECRIS nach Anspruch 2,
**dadurch gekennzeichnet, daß**
die Plasmabrennkammer (4) der ECRIS und das Rohrstück (8) mit Koaxialkabel (9) von einem dielektrischen Rohr (14) umgeben sind, um den jeweiligen, koaxial liegenden Solenoid (6) an den beiden Endbereichen der Plasmabrennkammer (4) zur Erzeugung des magnetischen Spiegelfelds für den axialen Einschluß elektrisch davon zu isolieren.

4. ECRIS nach Anspruch 3,
**dadurch gekennzeichnet, daß**
das dielektrische Rohr (14) an der Innenwand mit einer elektrisch leitenden Folie ausgekleidet ist, die über dielektrische Filmstreifen dazu auf Distanz gehalten wird und an keiner Stelle elektrisch leitend mit dem Sechspol (5) verbunden ist.

5. ECRIS nach Anspruch 4,
**dadurch gekennzeichnet, daß**
das dielektrische Rohr (14) aus Plexiglas ist.

6. ECRIS nach Anspruch 5,
**dadurch gekennzeichnet, daß**
die Plasmabrennkammer (4) aus einem Material besteht oder deren Innenwand mit einem Material beschichtet ist, das einen möglichst kleinen Rekombinationskoeffizienten, wie Pyrex, bezüglich der Molekülbildung aus den Atomen des über den Einlaß (7) zugeführten Gases hat.

7. Verfahren zum Betreiben der Elektron-Zyklotron-Resonanz-Ionen-Quelle, ECRIS, nach einem der Ansprüche 1 bis 6 zur Bereitstellung radioaktiver und nicht radioaktiver Atome, bestehend aus den Schritten:
das Plasma in der Vakuumkammer (4) wird aus Wasserstoff, H₂, und/oder Deuterium, D₂, erzeugt,
die Ionenquelle wird bei niedrigem Neutralgasdruck, H₂ und/oder D₂, und hohem Ionenanteil im Bereich der wirksamen magnetischen Flasche der ECRIS betrieben,
die für die Plasmabildung notwendige Mikrowellenleistung wird über ein konzentrisches, elektrisch leitendes und axial verschiebbares Rohrstück (8) um die Plasmabrennkammer (4) eingekoppelt, an dem die Mikrowellenzuleitung (9,10) endet, und das Rohrstück wird so lange axial verschoben, bis die maximale Feldstärke der eingekoppelten Mikrowelle im Bereich der magnetischen Resonanzfeldstärke liegt,
der in einem Halter (11) der Ionenquelle vorhandene Phosphor ist auf der Achse des magnetischen Spiegelfeldes im Bereich des einen magnetischen Flaschenhalses exponiert, der der Extraktionsöffnung der ECRIS im Bereich des andern magnetischen Flaschenhalses gegenüber liegt, woraus er durch die Wechselwirkung mit dem Plasma entnommen wird, um teilweise auf der Innenwand der Plasmakammer (4) zu kondensieren, chemisch mit dem Wasserstoff und/oder Deuterium zu gasförmigem Phosphorwasserstoff zu reagieren und zur Atomionenbildung der vorhandenen Phosphorisotope oder Molekülionenbildung der verschiedenen Phosphorwasserstoffe in den Reaktionsraum zurückfällt.

## Claims

1. Electron-Cyclotron-Resonance-Ion-Source = ECRIS (1), having an ion extraction device for an installation for doping vascular stents with radioactive and non-radioactive atoms, a plasma combustion chamber (4), a magnetic hexode (5) disposed concentrically around the plasma combustion chamber, and means for inputting microwave power into the plasma combustion chamber (4), **characterised in that**
a thin-walled and electrically conductive tubular piece (8) is situated coaxially between the cylindrical plasma combustion chamber (4) and the magnetic hexode (5) for inputting the microwave power and is axially displaceable on the plasma combustion chamber (4) for setting the optimum microwave input,
a coaxial cable (9), with which the microwave power is brought to an input aperture (10) in the wall of the tubular piece (8), is electrically conductively in contact there with its external casing, the internal conductor protrudes through the input aperture (10) in an insulated manner and forms a flat loop at the internal wall of the tube, the end of said loop in turn being in contact with the internal wall of the tube,
the plasma combustion chamber (4) is permeable to microwaves.

2. ECRIS according to claim 1, **characterised in that** the coaxial cable (9) for supplying microwaves is laid parallel to the axis (12) of the plasma combustion chamber (4) between two poles of the hexode (5).

3. ECRIS according to claim 2, **characterised in that** the plasma combustion chamber (4) of the ECRIS and the tubular piece (8) with coaxial cable (9) are surrounded by the dielectric tube (14) in order to insulate electrically therefrom the respective, coaxially lying solenoid (6) at the two end regions of the plasma combustion chamber (4) to produce the magnetic mirror field for the axial inclusion.

4. ECRIS according to claim 3, **characterised in that** the dielectric tube (14) is coated on the internal wall with an electrically conductive foil, which is kept apart from said wall via dielectric film strips and is at no point electrically conductively connected to the hexode.

5. ECRIS according to claim 4, **characterised in that** the dielectric tube (14) is formed from plexiglass.

6. ECRIS according to claim 5, **characterised in that** the plasma combustion chamber (4) is formed from a material, or the internal wall of said chamber is coated with a material, which has as low a recombination coefficient as possible, such as Pyrex, in respect of the formation of molecules from the atoms of the gas supplied via the inlet (7).

7. Method of operating the Electron-Cyclotron-Resonance-Ion-Source, ECRIS, according to one of claims 1 to 6 in order to prepare radioactive and non-radioactive atoms, said method comprising the steps:
the plasma in the vacuum chamber (4) is produced from hydrogen, H₂, and/or deuterium, D₂,
the ion source is operated with a low neutral gas pressure, H₂ and/or D₂, and with a high ion content in the region of the effective magnetic bottle of the ECRIS,
the microwave power, necessary for the plasma formation, is input via a concentric, electrically conductive and axially displaceable tubular piece (8) around the plasma combustion chamber (4), at which the microwave supply line (9, 10) terminates, and the tubular piece is axially displaced for such a distance until the maximum field strength of the input microwave lies within the range of the magnetic resonance field strength,
the phosphorus, which is present in a holder (11) of the ion source, is exposed on the axis of the magnetic mirror field in the region of the one magnetic bottle neck which lies opposite the extraction aperture of the ECRIS in the region of the other magnetic bottle neck, from which it is removed by the interaction with the plasma, in order to condense partially on the internal wall of the plasma chamber (4), to react chemically with the hydrogen and/or deuterium to form gaseous phosphorus hydride, and the phophorus falls back into the reaction chamber for the atom ion formation of the existing phosphorus isotopes or for the molecule ion formation of the various phosphorus hydrides in the reaction chamber.

## Revendications

1. Source ionique de résonance d'électrons de cyclotrons (ECRIS) comportant une installation d'extraction d'ions, pour une installation de dopage d'embouts de récipients avec des atomes radioactifs et non radioactifs, comprenant une chambre de combustion à plasma (4), un ensemble six-pôles magnétique (6) installé concentriquement dans la chambre de combustion à plasma, et des moyens pour injecter une puissance hyperfréquence dans la chambre de combustion à plasma (4),
**caractérisée en ce que**
pour injecter la puissance hyperfréquence, un tube (8) à paroi mince, conducteur électrique, est prévu coaxialement entre la chambre de combustion à plasma (4) cylindrique et l'ensemble à six pôles magnétiques (5), et ce tube est coulissant axialement sur la chambre de combustion à plasma (4) pour régler le couplage d'injection hyperfréquence optimum,
un câble coaxial (9) fournissant la puissance hyperfréquence à un orifice d'injection (10) réalisé dans la paroi du morceau de tube (8) en y étant en contact électrique avec son enveloppe extérieure, le conducteur intérieur traversant de manière isolée l'orifice d'injection (10) et formant une boucle plate contre la paroi intérieure du tube, boucle dont l'extrémité est de nouveau en contact avec la paroi intérieure du tube,
la chambre de brûleur à plasma (4) étant perméable aux hyperfréquences.

2. Source (ECRIS) selon la revendication 1,
**caractérisée en ce que**
le câble coaxial (9) pour guider les hyperfréquences est parallèle à l'axe (12) de la chambre de combustion à plasma (4) entre les deux pôles de l'ensemble six-pôles (5).

3. Source (ECRIS) selon la revendication 2,
**caractérisée en ce que**
la chambre de combustion à plasma (4) de la source ECRIS et l'élément de tube (8) avec le câble coaxial (9) sont entourés par un tube diélectrique (14), pour isoler électriquement de l'injection axiale le solénoïde (6) respectif, installé coaxialement au niveau de chacune des deux zones d'extrémité de la chambre de combustion à plasma (4), pour générer le champ magnétique miroir de l'injection axiale.

4. Source (ECRIS) selon la revendication 3,
**caractérisée en ce que**
le tube diélectrique (14) a une paroi intérieure revêtue d'un film conducteur électrique, qui est maintenu à distance par une bande de film diélectrique et qui n'est relié en aucun endroit, de façon électroconductrice à l'ensemble avec l'ensemble six-pôles (5).

5. Source (ECRIS) selon la revendication 4,
**caractérisée en ce que**
le tube diélectrique (14) est en Plexiglas.

6. Source (ECRIS) selon la revendication 5,
**caractérisée en ce que**
la chambre de combustion à plasma (4) est en un matériau ou encore sa paroi intérieure est munie d'une matière de revêtement ayant un coefficient de recombinaison aussi réduit que possible comme du Pyrex vis-à-vis de la formation de molécules à partir des atomes du gaz arrivant par l'entrée (7).

7. Procédé de mise en oeuvre d'une source d'ions à résonance électronique à cyclotrons (ECRIS) selon l'une des revendications 1 à 6, pour fournir des atomes radioactifs et des atomes non radioactifs, comprenant les étapes suivantes :
le plasma est formé dans la chambre à vide (4) à partir d'hydrogène H₂ et/ou de deutérium D₂,
la source d'ions fonctionne avec une faible pression de gaz neutre H₂ et/ou D₂ et une forte teneur en ions dans la plage de la bouteille magnétique active de la source (ECRIS),
la puissance de micro-ondes nécessaire au développement du plasma est injectée autour de la chambre de combustion à plasma (4) par un élément tubulaire (8) conducteur électrique, concentrique et coulissant axialement, élément de tube où s'arrête la conduite d'alimentation hyperfréquence (9, 10), et l'élément tubulaire est coulissé axialement jusqu'à ce que l'intensité de champ maximale des hyperfréquences injectées se situe au niveau de l'intensité du champ de résonance magnétique,
le phosphore existant dans un support (11) de la source d'ions est exposé suivant l'axe du champ magnétique miroir dans la zone d'un col de bouteille magnétique situé en regard de l'orifice d'extraction de la source (ECRIS) au niveau de l'autre col de bouteille magnétique, en étant entraîné par le plasma par cet effet d'alternance pour se condenser en partie sur la paroi intérieure de la chambre à plasma (4), réagir chimiquement avec l'hydrogène et/ou le deutérium pour donner de l'acide phosphorique gazeux, et se précipiter dans la chambre de réaction pour former les ions atomiques des isotopes de phosphore présents ou les ions moléculaires des différents acides de phosphore.
